(19) Europäisches Patentamt
European Patent Office
Office européen des brevets

(11) Numéro de publication: 0 274 324 B1

# (12) FASCICULE DE BREVET EUROPEEN

(45) Date de publication du fascicule du brevet: **06.02.91**

(51) Int. Cl.$^5$: **C 07 D 333/20**

(21) Numéro de dépôt: **87402926.7**

(22) Date de dépôt: **18.12.87**

## (54) Procédé de préparation de thiényléthylamines et dithiényléthylamines ainsi obtenues.

(30) Priorité: **23.12.86 FR 8618101**

(43) Date de publication de la demande: **13.07.88 Bulletin 88/28**

(45) Mention de la délivrance du brevet: **06.02.91 Bulletin 91/06**

(84) Etats contractants désignés: **AT BE CH DE ES FR GB GR IT LI LU NL SE**

(56) Documents cités:
**DE-A-2 623 174**
**CHEMICAL ABSTRACTS, vol. 75, no. 21, 22 novembre 1971, page 250, résumé no. 129308j, Columbus, Ohio, US; P. POPESCU et al.: "Preparation of ethylamines from acetonitrile. 1. Laboratory study of the preparation of a selective catalyst for monoethylamine", & REV. CHIM. (BUCHAREST) 1971, 22(6), 327-31**
**CHEMICAL ABSTRACTS, vol. 77, no. 19, 6 novembre 1972, page 344, résumé no. 125951g, Columbus, Ohio, US; & RO-A-53 953 (INSTITUTUL "CHIMIGAZ") 29-10-1971**

(73) Titulaire: **SANOFI**
**40, Avenue George V**
**F-75008 Paris (FR)**

(72) Inventeur: **Radisson, Joel**
**10, avenue Winston Churchill**
**F-31100-Toulouse (FR)**
Inventeur: **Braye, Emile**
**Le Puntis Lagardelle sur Lèze**
**F-31190 Auterive (FR)**

(74) Mandataire: **Moncheny, Michel et al**
**c/o Cabinet Lavoix 2 Place d'Estienne d'Orves**
**F-75441 Paris Cedex 09 (FR)**

(56) Documents cités:
**CHEMICAL ABSTRACTS, vol. 89, no. 9, 28 aou7t 1978, page 395, résumé no. 75245e, Columbus, Ohio, US; & JP-A-7 850 109 (ASAHI CHEMICAL INDUSTRY CO., LTD) 08-05-1978**

EP 0 274 324 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention est relative à un procédé pour la préparation de thiényléthylamines.

Plus particulièrement, l'invention se rapporte à une nouvelle voie de synthèse de thiényléthylamines représentées par la formula:

R—NH—R'    I

dans laquelle R représente le groupe

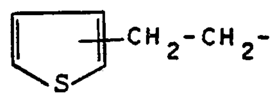

et R' représente un atome d'hydrogéne où un groupe identique à R.

Cette nouvelle voie consiste en l'hydrogènation catalytique de nitriles de structure générale II.

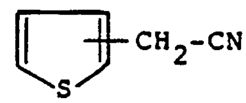

II

Les composés de formule I dans laquelle R' est l'hydrogène sont bien connus dans la littérature et sont utilisés comme intermédiares dans la préparation de dérivés employés aussi bien dans l'industrie chimique que dans l'industrie pharmaceutique (par exemple FR—A—2 397 417 et 2 358 150).

Les composés de formule I dans laquelle R' représente un groupe identique à R sont nouveaux et constituent un autre aspect de la présente invention.

Les procédés pour la préparation des composés de formule I ou R' est l'hydrogène sont décrits dans la littérature, par exemple:

réduction de béta-nitrovinyl-2 ou -3 thiophènes à l'aide de l'hydrure double de lithium et d'aluminium (S. GRONOWITZ et E. SANDBERG, ARKIV För Kem, 1970, *32,* 217—227) ou par voie électrolytique (FR—A—2 415 671);

dégradation de CURTIUS sur la (thiényl-3)-3 proprionazide (E. CAMPAIGNE et W. C. McCARTY, J. Am. Chem. Soc. 1954, 76, 4466—4467);

dégradation d'HOFFMANN du (thiényl-2)-3 propionamide (G. BARGER et A. P. T. EASSOU, J. CHem. Soc. 1938, 2100—2104);

amination des haloéthyl-2 ou arylsulfonyloxyéthyl-2 thiophènes soit directe (F. F. BLICKE et J. H. BURCKHALTER, J. Am. Soc. 9142, 64, 477—480) soit par l'intermédiaire d'un phtalimide (FR—A—2 299 332).

Toutes ces méthodes sont difficiles à mettre en ouevre au plan industriel soit à cause des matières premières difficilement accessibles, comme dans le cas des propionazides ou amides les dégradations de CURTIUS et de HOFFMANN, soit à cause des réactifs coûteux et/ou dangereux à utiliser, comme dans le cas de l'hydrure de lithium et l'aluminium, soit à cause des rendements insuffisants.

Il est également connu que la (thiényl-2)-2 éthylamine et la (thiényl-3)-2 éthylamine peuvent être préparées par réduction de thiophèneacétonitriles avec l'hydrure de lithium et d'aluminium (E. COMPAIGNE et W. C. McCARTY, J. Am. Chem. Soc. 1954, *76,* 4466—4467 ou avec du sodium dans le butanol à reflux (F. F. BLICKE et J. H. BURCHHALTER, J. Am. Chem. Soc. 1942, *64,* 477—480), mais ces méthodes aussi présentent les inconvénients indiqués ci-dessus.

La (thienyl-2)-2 éthylamine a été obtenue par réduction électrochimique du thiophène-2-acétonitrile avec un rendement maximal de 25% seulement (W. HERZ et L. TSAI, J. Am. Chem. Soc. 1955, *77,* 3529—3531).

L'hydrogénation catalytique du thiénylacétonitrile n'a jamais été décrite et, il est connu que le soufre en général, et le thiophène, en particulier inactivent, dans une large mesure, tous les catalyseurs d'hydrogénation.

Les catalyseurs au nickel, par exemple, sont rapidement empoisonnés (KUBOTA et Al. Jap. J. Chem. 2, 45, 1925) et même les catalyseurs sulfurés connus pour être les plus résistants à l'empoisonnement, deviennent vite inactifs. Ainsi, avec le disulfure de molybdène, à 200°C et sous 200 atmosphères, le taux de conversion de thiophène en thiolane reste faible. (CAWLEY et Al. J. Soc. Chem. Ind. *62,* 116 (1943).

MOZIMGO (J. Am. Chem. Soc. *67,* 2092 (1945) a pu effectuer la transformation du thiophène en thiolane avec un rendement de 70%, mais en utilisant 200% de palladium par rapport au substrat, ce qui interdit la transposition de cette méthode au stade industriele.

Il est connu encore que le nickel de Raney décompose le thiophène donnant de l'hydrogène sulfuré et du butane à 80°C dans l'éthanol (H. HAUPTMANN et Al. Chem. Rev. *62,* 347 (1962), et "Thiophene and its derivatives" par H. D. HARTOUGH, p. 167 et 168, (1952), Interscience Publishers N.Y., dans la serie "The Chemistry of Heterocyclic Compounds").

Enfin, il est connu (L. Kh FREIDLIN et E. F. LITVIN, KHIMIYA G. et SOED, 1967, *3,* 22), que l'hydrogénation catalytique du béta-nitrovinyl-2 thiophène ne donne que des traces de thiényléthylamine.

Une telle réaction fait également l'object de FR—A—2 508 456 au nom de la Demanderesse.

On a maintenant trouvé qu'en utilisant un catalyseur à base de nickel ou de cobalt il est possible de soumettre un thiophèneacétonitrile à une hydrogénation pour obtenir des thiényléthylamines avec des rendements très élevés.

On a également trouvé que par ce type d'hydrogénation on obtient une thiényléthylamine primaire et/ou thiényléthylamine secondaire et que la réaction peut être dirigée vers l'obtention de l'une ou l'autre des deux amines ou bien vers un mélange de thiényléthylamine et de di(thiényléthyl)amine facilement séparables.

Ainsi, la présente invention a pour objet un procédé pour la préparation de thiényléthylamines de formule I suivante et de leurs sels d'addition avec des acides,

$$R—NH—R' \qquad I$$

dans laquelle R représente le groupe

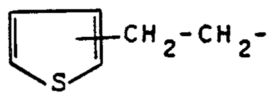

et R' représente un atome d'hydrogéne où un groupe identique à R caractérisé en ce que l'on soumet un thiénylacétonitrile de formule

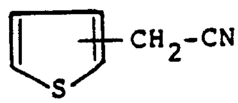

II

en solution, à une hydrogénation en présence d'un catalyseur à base de nickel ou de cobalt, à une température comprise entre 15 et 80°C et une pression entre la pression ambiante et 100 bars (entre $10^5$ et $10^7$ Pa) et l'on transforme, éventuellement les produits ainsi obtenus dans leurs sels d'addition.

La pression peut être de préférence comprise entre 1 et 6 MPa.

Les catalyseurs qui conviennent pour cette hydrogénation sont notamment le nickel de Raney, le borure de nickel (R. Paul et al., Ind. Eng. Chem. *44* (5) 1006 (1952), les catalyseurs au nickel sur support inerte, le nickel suivant URUSHIBARA (K. HATA, URUSHIBARA Catalyst, University of Tokyo Press, Tokyo 1971) qui est du nickel precipité à partir d'une solution d'un de ses sels par un métal electropositif tel que Al ou Zn, et les catalyseurs similaires au cobalt.

Les quantités de catalyseur mises en oeuvre sont de l'ordre de 2 à 30% en poids par rapport au substrat.

La concentration du substrat peut varier dans de grandes proportions; pour des raisons économiques on opère à des concentrations comprises entre 20 et 40% (p/v).

Le substrat peut être introduit dans le milieu réactionnel, en une seule fois ou au fur et à mesure de l'avancement de la réaction.

Le solvant est tout solvant organique classique ou mélange de solvant, anhydre ou aqueux, capable de dissoudre le nitrile, qui ne s'hydrogène pas au cours de la réaction et qui ne conduit pas à des produits secondaires indésirables au point de compromettre l'aspect économique du procédé.

Le solvant sera de préférence un alcool, particulièrement un alcool léger tel que le méthanol ou l'éthanol ou un alcoxy-e-éthanol ou-2-propanol ou similaire ou bien on peut opérer en milieu hydroalcoolique. Convenient également les éthers tels que le tétrahydrofuranne, le dioxanne, les éthers de l'éthylèneglycol ou d'autres glycols, les éthers aliphatiques.

Le milieu réactionnel peut utilement être rendu acide par un solvant. Le solvent peut alors être un acide carboxylique, pur ou en mélange avec un autre solvant, dans la mesure où il n'attaque pas le catalyseur, notamment un mélange d'acide acétique et d'un alcool aliphatique en $C_1$—$C_4$ comme l'éthanol, par exemple en une proportion de 75—25.

Lorsque la quantité calculée d'hydrogène est absorbée, on distille la thiényléthylamine primaire qui passe à une température inférieure et, ensuite, on augmente la température et l'on distille la di(thiényléthyl)amine. On peut aussi séparer les deux amines sur la base des solubilités différentes de leur seul. Ainsi, le chlorhydrate de (thiényl-2)-2-éthylamine est très soluble dans l'eau, celui de la di(thiényl-2 éthyl)amine est insoluble.

Pour favoriser la formation des thiényléthylamines primaires, on effectue l'hydrogénation en présence d'une base telle qu'un hydroxyde de métal alcalin, notamment NaOH, LiOH, ou alcalino-terreux, l'ammoniac, un hydroxyde d'ammonium quaternaire de formule $HON(R_1)_4$ dans laquelle $R_1$ représente un alkyle en $C_1$—$C_4$, un carbonate de métal alcalin tel que le carbonate de sodium, de potassium ou de césium, en des quantités variant de 0,1% à 15% par rapport au thiénylacétonitrile mis en oeuvre. De cette façon, on peut diriger l'hydrogénation vers l'obtention de l'amine primaire avec des rendements supérieurs à 80%. Dans certains cas, an choisissant opportunément la base, il est possible d'obtenir presque exclusivement l'amine primaire.

Les thiényléthylamines ainsi obtenues peuvent être transformées dans leurs sels d'addition par

traitement d'une solution de l'amine avec l'acide dissous de préférence dans le même solvant.

La présente invention permet donc la préparation à l'échelle industrielle des thiényléthylamines primaires, par exemple, la (thiényl-2)-2-éthylamine, selon un procédé très simple et peu onéreux, en regard de la technique antérieure et rend donc accessibles des intermédiaires de synthèse très utiles surtout dans l'industrie pharmaceutique.

La présente invention permet également l'obtention des thiényléthylamines secondaires, par exemple la di(thiényl-2)-2 éthyl) amine et la di(thiényl-3)-2 éthyl)amine, qui n'ont jamais été isolées, et leurs sels.

Ainsi, selon un autre aspect, la présente invention concerne les composés de formule (I), dans laquelle R' est un groupe identique à R et leurs sels d'addition.

Ces produits sont, aux aussi, des intermédiaires utiles pour la préparation de composés ayant des propriétés pharmacologiques intéressantes.

Par exemple, par alkylation suivi d'une quaternisation, on obtient des composés ayant une activité anti-bactérienne.

Les exemples non limitatifs suivants, sont Ordonnés à titre d'illustration de la présente invention.

## Exemple 1

(Thiényl-2)-2 éthylamine

Dans un hydrogénateur de 500 ml, à agitation magnétique, on introduit 50 g de thiényl-2-acétonitrile, 200 ml d'éthanol, 2,5 ml d'hydroxyde de sodium 10N et 10 g de nickel de Raney. La réaction d'hydrogénation s'effectue à 50°C sous pression de 30 bars (3 MPa).

Le catalyseur est ensuite filtré, l'alcool évaporé et la thiényléthylamine distillée à 90°C/15 mm Hg (2 kPa).

On obtient ainsi 38 g du produit recherché (Rendement 74%) dont les propriétés correspondent à celles données dans la littérature.

## Exemple 2

(Thiényl-2)-2 éthylamine

Selon le procédé décrit dans l'exemple 1, en hydrogénant 50 g de thiényl-2 acétonitrile dans 200 ml d'éthanol, en présence de 10 g de nickel de Raney, de 2,5 ml d'eau et 3,5 g $K_2CO_3$, sous une pression comprise entre 15 et 22 bars (1,5 et 2,2 MPa), on obtient 44,95 g de (thiényl-2)-2 éthylamine. Rendement 87%.

## Exemple 3

(Thiényl-2)-2 éthylamine

Selon le procédé décrit à l'exemple 1, en introduisant dans l'hydrogénateur 50 g de thiényl-2 acétonitrile, 200 ml d'éthanol, 5 ml d'eau, 12,5 g d'hydroxyde de tétraméthylammonium en solution à 20% dans le méthanol et 10 g de nickel de Raney, sous une pression comprise entre 26 et 40 bars (2,6 et 4 MPa), on obtient 32,5 g de (thiényl-2)-2 éthylamine. Rendement 65%.

## Exemple 4

(Thiényl-2)-2 éthylamine

Selon le procédé décrit à l'exemple 1, en introduisant dans l'hydrogénateur 50 g de thiényl-2 acétonitrile, 200 ml d'éthanol, 10 ml d'eau, 5 g d'hydroxyde de lithium et 10 g de nickel de Raney, sous une pression comprise entre 26 et 40 bars (2,6 et 4 MPa), on obtient 35,9 g de (thiényl-2)-2 éthylamine. Rendement 69,7%.

## Exemple 5

(Thiényl-2)-2 éthylamine

Selon le procédé décrit à l'exemple 1, on introduit dans l'hydrogénateur 50 g de thiényl-2 acétonitrile, 200 ml d'isopropanol, 5 ml d'eau, 4 g de carbonate de potassium et 10 g de nickel de Raney, sous pression comprise entre 28 et 40 bars (2,8 et 4 MPa), on obtient 40,7 g de (thiényl-2)-2 éthylamine. Rendement 79%.

## Exemple 6

(Thiényl-2)-2 éthylamine

Dans un autoclave d'hydrogénation de 500 ml on introduit 50 g de thiényl-2 acétonitrile, 200 ml de méthanol, 10 g de nickel de Raney et 1 g d'hydroxyde de sodium. Après hydrogénation à 50°C et sous une pression comprise entre 18 et 40 bars (1,8 et 4 MPa), selon la procédure de l'exemple 1, on distille 41,8 g de (thiényl-2-)-2 éthylamine. Rendement 81%.

## Exemple 7

(Thiényl-2)-2 éthylamine

Selon le procédé décrit dans l'exemple 1, en hydrogénant 50 g de thiényl-2 acétonitrile dans 150 ml d'éthoxy-2 éthanol, en présence de 10 g de nickel de Raney et de $K_2CO_3$, sous pression comprise entre 26 et 50 bars (2,6 et 5 MPa), on obtient 32,1 g de thiényl-2)-2 éthylamine. Rendement 62,5%.

Exemple 8

(Thiényl-2)-2 éthylamine

Selon le procédé décrit dans l'exemple 1, en hydrogénant 50 g de thiényl-2 acétonitrile dans 150 ml d'éthanol, en présence de 10 g de nickel de Raney et de 2,5 ml de lessive de potasse à 40% sous pression comprise entre 15 et 40 bars (1,5 et 4 MPa), on obtient 25,4 g de (thiényl-2)-2 éthylamine. Rendement 49%.

Exemple 9

(Thiényl-2)-2 éthylamine

Selon le procédé décrit dans l'exemple 1, en hydrogénant 50 g de thiényl-2 acétonitrile dans 200 ml d'éthanol, en présence de 10 g de nickel de Raney et de 6 g de $Cs_2CO_3$, sous pression comprise entre 23 et 50 bars (2,3 et 5 MPa), on obtient 26,5 g de (thiényl-2)-2 éthylamine. Rendement 51,5%.

Exemple 10

(Thiényl-2)-2 éthylamine

Dans un autoclave de 500 ml, on introduit 80 g de thiényl-2 acétonitrile, 120 ml d'éthanol, 16 g de nickel de Raney et 4 ml de lessive de soude 10N. Après hydrogénation à 50°C, sous une pression comprise entre 20 et 40 bars (2 et 4 MPa), le catalyseur est filtré, le solvant évaporé. La (thiényl-2)-2 éthylamine est distillée à 90°C/15 mm Hg (2 kPa). On obtient 46,6 g de produit attendu. Rendement 56,5%.

Exemple 11

(Thiényl-2)-2 éthylamine

Dans un autoclave de 500 ml, on introduit 50 g de thiényl-2 acétonitrile 200 ml d'éthanol, 10 g de nickel de Raney, 10 ml d'eau et 5 g de $K_2CO_3$. Le mélange est hydrogéné à 50°C sous une pression constante de 3 bars (300 kPa) jusqu'à cessation de l'absorption d'hydrogène.

L'analyse du milieu réactionnel par chromatographie montre un rendement en (thiényl-2)-2 éthylamine de 79%.

Exemple 12

(Thiényl-2)-2 éthylamine

Selon le procédé décrit dans l'exemple 1, en hydrogénant 50 g de thiényl-2 acétonitrile dans 200 ml d'éthanol, en présence de 10 g de cobalt URUSHIBARA et sous pression comprise entre 14 et 40 bars (1,4 et 4 MPa), on obtient 31 g de (thiényl-2)-2 éthylamine. Rendement 60%.

Exemple 13

(Thiényl-2)-2 éthylamine

Selon le procédé décrit dans l'exemple 1, en hydrogène 50 g de thiényl-2 acétonitrile dans 100 ml de méthanol contenant 100 ml d'ammoniac liquide, en présence de 10 g de nickel de Raney. L'hydrogénation est conduite à 50°C sous une pression comprise entre 31 et 55 bars (3,1 et 5,5 MPa), jusqu'à absorption de la quantité théorique d'hydrogène. On obtient 37,8 g de (thiényl-2)-2 éthylamine. Rendement 74%.

Exemple 14

(Thiényl-2)-2 éthylamine

Dans un autoclave d'hydrogénation de 500 ml on introduit 50 g de thiényl-2 acétonitrile, 200 ml d'un mélange d'éthanol et d'acide acétique dans un rapport 1 à 3 et 10 g de nickel de Raney. Après hydrogénation à 50°C, sous pression comprise entre 8 et 32 bars (0,8 et 3,2 MPa), le catalyseur est filtré, le solvant évaporé. Le résidu est repris à l'eau, basifié par de la soude et extrait à l'éther. L'éther est évaporé et la (thienyl-2)-2 éthylamine distillé. On obtient 26,3 g de produit attendu. Rendement 51%.

Exemple 15

(Thiényl-2)-2 éthylamine

Selon le procédé décrit dans l'exemple 1, on hydrogène 18,7 g de thiényl-2 acétonitrile dans un mélange d'acide acétique (225 ml) et d'éthanol (75 ml) en présence de 1,8 g de $N_2B$. La réaction s'effectue à 65°C sous pression comprise entre 24 et 31 bars (2,4 et 3,1 MPa). Lorsque l'absorption d'hydrogène s'arrête, la chromatographie montre un rendement de 75%.

Exemple 16

(Thiényl-3)-2 éthylamine

Dans un autoclave de 500 ml on introduit 50 g de thiényl-3 acétonitrile, 200 ml d'éthanol, 2,5 ml de lessive de soude 10N et 10 g de nickel de Raney. Le mélange est hydrogéné à 50°C, sous une pression comprise entre 22 et 40 bars (2,2 et 4 MPa), jusqu'à absorption de la quantité théorique en hydrogène. Le catalyseur est ensuite filtré, l'alcool évaporé et la (thienyl-3)-2 éthylamine distillée à 95—100°C/15 mm Hg. On obtient ainsi, 41,6 g de produit attendu. Rendement 81%.

Exemple 17

(Thiényl-3)-2 éthylamine

Dans un autoclave d'hydrogénation de 500 ml, on introduit 50 g de thiényl-3 acétonitrile, 200 ml d'éthanol, 5 ml d'eau, 10 g de nickel de Raney et 3 g de $K_2CO_3$. Le mélange est hydrogéné à 50°C, sous pression comprise entre 20 et 40 bars (2 et 4 MPa), jusqu'à absorption de la quantité théorique en hydrogène. Le catalyseur est filtré, le solvant évaporé et la (thiényl-3)-2 éthylamine distillée à 95—100°C/15 mm Hg. On obtient ainsi 35 g de produit attendu. Rendement 68%.

Exemple 18

Chlorhydrate de di((thiényl-2)éthyl)amine

Dans un autoclave de 500 ml on introduit 50 g de thiényl-2 acétonitrile, 200 ml d'éthanol et 10 g de nickel de Raney. Le mélange est hydrogéné à 50°C, sous pression comprise entre 17 et 50 bars (1,7 et 5 MPa), jusqu'à absorption de la quantité théorique en hydrogène. Le catalyseur est filtré, le solvent évaporé et le résidu est repris par 200 ml d'acide chlorhydrique 2N. Le chlorhydrate de l'amine secondaire insoluble est filtré, rincé à l'acétone et séché à 50°C poids constant. On obtient 43,2 g de chlorhydrate de di((thiényl-2)-2 éthyl) amine, PF = 244°C avec décomposition à partir de 220°C, analyse élémentaire correcte. Rendement 79%.

Exemple 19

Di((thiényl-3)-2 éthy)l amine et (thiényl-3)-2 éthylamine

Dans un autoclave de 500 ml on introduit 50 gde thiényle-3 acétonitrile, 200 ml d'éthanol et 10 g de nickel de Raney. On hydrogène à 50°C sous une pression comprise entre 20 et 40 bars (2 et 4 MPa), jusqu'à absorption de la quantité théorique en hydrogène. Le catalyseur est filtré, le solvant évaporé et le résidu est distillé à la trompe à eau puis la pompe à palette. On obtient 4,5 g de (thiényl-3-)-2 éthylamine qui distille à 95—100°C sous 15 mm de Hg (rendement 9%) et 37,05 g de di((thiényl-3)-2 éthyl)amine qui distille à 128—135°C/3 mm Hg (400 Pa) (rendement 78%). La di((thiényl-3)-2 éthyl) amine est caractérisée par RMN du proton (=2,8, 6,8, 7,1 ppm, NH à 1,1 ppm dans $CDCl_3$) et du carbone 13 ($-CH_2-CH_2-N$ à 30,7 ppm, $-CH_2-CH_2-N$ à 50,1 ppm, méthines du thiophène à 121, 125 et 128 ppm, carbone substitué en position 3 à 140,4 ppm, dans $CDCl_3$).

Exemple 20

(Thiényl-3)-2 éthylamine et di((thiényl-3)-2 éthylamine

Selon le procédé décrit dans l'exemple 19, on hydrogène à 50°C, 50 g de thiényl-3 acétonitrile dans 200 ml de dioxanne, en présence de 10 g de nicklel de Raney, sous un pression comprise entre 21 et 56 bars (2,1 et 5,6 MPa), jusqu'à absorption de la quantité théorique en hydrogène. On obtient ainsi 38,3 g de di((thiényl-3)-2 éthylamine (rendement 80,5%) et 6,6 g de (thiényl-3)-2 éthylamine. Rendement 13%.

Exemple 21

(Di((thiényl-3)-2 éthyl)amine et (thiényl-3)-2 éthylamine

Selon le procédé décrit dans l'exemple 19, on hydrogène 50 g de thiényl-3 acétonitrile dans 200 ml de tétrahydrofuranne, en présence de 7 g de triéthylamine et de 10 g de nickel de Raney, sous une pression comprise entre 20 et 40 bars (2 et 4 MPa). On obtient ainsi 35 g de di((thiényl-3)-2 éthylamine (rendement 74%) et 9,4 g de (thiényl-3)-2 éthylamine. Rendement 18%.

Exemple 22

Di((thiényl-2)-2 éthyl)amine

En procédant comme dans l'exemple 19, en hydrogénant 50 g de thiényl-2 acétonitrile dans 200 ml d'éthanol en présence de nickel de Raney, sous une pression comprise entre 20 et 40 bars (2 et 4 MPa), on obtient 38 g de di((thiényl-2)-2 éthyl)amine. Eb: 125—130°C mm Hg. Rendement 80%.

Exemple 23

(Thiényl-2)-2 éthyl)amine et di((thiényl-2)-2 éthyl)amine

En procédant comme dans l'exemple 1, en hydrogénant 50 g de thiényl-2 acétonitrile dans 200 ml d'éthanol à 95% aqueux, en présence de 3 g de $K_2CO_3$ et de 10 g de Nickel de Raney, sous pression constante de 12 bars (1,2 MPa) et à 25°C, on obtient 40,3 g de (thiényl-2)-2 éthylamine. Rendement 78,0%.

Exemple 24

En procédant comme dans l'exemple 1, en hydrogénant 50 g de thiényl-2 acétonitrile dans 200 ml d'éthanol à 95% aqueux, en présence de 3 g de $K_2CO_3$ et de 5 g de Nickel de Raney, sous pression constante de 20 bars (2 MPa) et à 40°C, on obtient 39,9 g de (thiényl-2)-2 éthylamine. Rendement 77%.

Exemple 25

Dans un hydrogénateur de 3,5 l, on introduit 0,6 l d'éthanol à 95% aqueux. La pression d'hydrogène est établie à 20 bars (2 MPa) et la température à 50°C. On introduit à l'aide d'une pompe doseuse, en 8 h, une solution de 200 g de thiényl-2 acétonitrile dans 1 l d'éthanol à 95% aqueux.

Lorsque toute absorption d'hydrogène a cessé, le catalyseur est filtré, le solvant évaporé et la (thiényl-2)-2 éthylamine est distillée. On obtient 146,7 g de produit attendu. Rendement 71%.

**Revendications**

1. Procédé de préparation de thiényléthylamines de formule

R—NH—R'

dans laquelle R représente

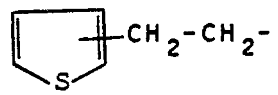

et R' représente H ou le radical R
caractérisé en ce que l'on soumet une solution d'un thiényl-acétonitrile de formule

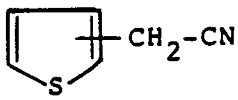

à une hydrogénation, en présence d'un catalyseur à base de nickel ou de cobalt, à une pression comprise entre $10^5$ et $10^7$ Pa et à une température comprise entre 15°C et 80°C.

2. Procédé selon la revendication 1, caractérisé en ce que la réaction est effectuée dans un alcool aliphatique.

3. Procédé selon les revendications 1 ou 2, caractérisé en ce que le catalyseur est le nickel de Rany.

4. Procédé selon la revendication 3, caractérisé en ce que la concentration du nitrile dans le milieu réactionnel est comprise entre 20 et 40% (p/v) et celle du catalyseur entre 2 est 30% (p/p) par rapport au substrat.

5. Procédé selon l'une quelconque des revendications 1 à 4, caractérisé en ce que la pression est comprise entre 1 et 6 MPa.

6. Procédé selon l'une quelconque des revendications 3 à 5, caractérisé en ce que l'hydrogénation est effectuée en présence d'une base minérale ou organique.

7. Procédé selon la revendication 6, caractérisé en ce que la base est choisi parmi NaOH, LiOH et $K_2CO_3$.

8. Procédé selon la revendication 6, caractérisé en ce que la base est choisi parmi $NH_3$, KOH, $Cs_2CO_3$ et HON $(R_1)_4$ dans laquelle $R_1$ représente un alkyle en $C_1$ à $C_4$.

9. Procédé selon l'une quelconque des revendications 6 à 8, caractérisé en ce que la base est utilisée en des quantités variant de 0,1 à 15% par rapport à la quantité de thiényl-acétonitrile mise en oeuvre.

10. Procédé selon l'une quelconque des revendications 1 à 3, caractérisé en ce que le catalyseur est le cobalt de URUSHIBARA, qui est du cobalt précipité à partir d'une solution d'un de ses sels par un metal électropositif.

11. Procédé selon la revendication 10, caractérisé en ce que la pression est comprise entre 1 et 6MPa.

12. Procédé selon la revendication 1, caractérisé en ce que le milieu réactionnel est rendu acide par un solvant.

13. Procédé selon la revendication 12, caractérisé en ce que le solvant est un mélange d'un alcool aliphatique en $C_1$ à $C_4$ et d'acide acétique.

14. Procédé selon la revendication 13, caractérisé en ce que catalyseur est choisi parmi le nickel de Raney et le borure de nickel.

15. Procédé selon l'une des revendications 1 à 14, caractérisé en ce que les amines primaires et secondaires sont séparées par distillation.

16. Procédé selon l'une des revendications 1 à 14, caractérisé en ce que les amines primaires et secondaires sont séparées par précipitation de chlorhydrate de l'amine secondaire en milieu aqueux.

17. La di((thiényl-2)-2 éthyl)amine et son chlorhydrate.

18. La di((thiényl-3)-2 éthyl)amine et son chlorhydrate.

**Patentansprüche**

1. Verfahren zur Herstellung von Thienylethylaminen der Formel

R—NH—R'

in der R eine Gruppe

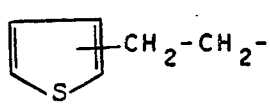

und R' H oder die Gruppe R bedeuten, gekennzeichnet durch Hydrierung einer Lösung eines Thienylacetonitrils der Formel

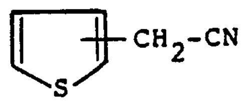

in Gegenwart eines Katalysators auf der Basis von Nickel oder Cobalt bei einem Druck von $10^5$ bis $10^7$ Pa und einer Temperatur von 15 bis 80°C.

2. Verfahren nach Anspruch 1, dadurch gekennzeichnet, daß die Reaktion in einem aliphatischen Alkohol vorgenommen wird.

3. Verfahren nach Anspruch 1 oder 2, dadurch gekennzeichnet, daß als Katalysator Raney-Nickel verwendet wird.

4. Verfahren nach Anspruch 3, dadurch gekennzeichnet, daß die Konzentration des Nitrils im Reaktionsmedium 20 bis 40% (G/V) und die Konzentration des Katalysators 2 bis 30% (G/G), bezogen auf das Substrat, betragen.

5. Verfahren nach einem der Ansprüche 1 is 4, dadurch gekennzeichnet, daß der Druck im Bereich von 1 bis 6 MPa liegt.

6. Verfahren nach einem der Ansprüche 3 bis 5, dadurch gekennzeichnet, daß die Hydrierung in Gegenwart einer anorganischen oder organischen Base vorgenommen wird.

7. Verfahren nach Anspruch 6, dadurch gekennzeichnet, daß die Base unter NaOH, LiOH und $K_2CO_3$ ausgewählt wird.

8. Verfahren nach Anspruch 6, dadurch gekennzeichnet, daß die Base unter $NH_3$, KOH, $Cs_2CO_3$ und $HON(R_1)_4$, wobei $R_1$ $C_1$—$C_4$-Alkyl bedeutet, ausgewählt wird.

9. Verfahren nach einem der Ansprüche 6 bis 8, dadurch gekennzeichnet, daß die Base in einer Menge von 0,1 bis 15%, bezogen auf die Menge des eingesetzten Thienylacetonitrils, angewandt wird.

10. Verfahren nach einem der Ansprüche 1 bis 3, dadurch gekennzeichnet, daß als Katalysator Urushibia-Cobalt verwendet wird, as einer Lösung eines Cobalt-salzes durch ein elektropositives Metall abgeschiedenes Cobalt ist.

11. Verfahren nach Anspruch 10, dadurch gekennzeichnet, daß der Druck im Bereich von 1 bis 6 Ma liegt.

12. Verfahren nach Anspruch 1, dadurch gekennzeichnet, daß das Reaktionsmedium mit einem Lösungsmittel angesäuert wird.

13. Verfahren nach Anspruch 12, dadurch gekennzeichnet, daß das Lösungsmittel ein Gemisch eines aliphatischen $C_1$—$C_4$-Alkohols mit Essigsäure ist.

14. Verfahren nach Anspruch 13, dadurch gekennzeichnet, daß der Katalysator unter Raney-Nickel und Nickelborid ausgewählt wird.

15. Verfahren nach einem der Ansprüche 1 bis 14, dadurch gekennzeichnet, daß die primären und sekundären Amine durch Destillation getrennt werden.

16. Verfahren nach einem der Ansprüche 1 bis 14, dadurch gekennzeichnet, daß die primären und sekundären Amine durch Ausfällung des Hydrochlorids des sekundären Amins in einem wäßrigen Medium getrennt werden.

17. Di-[2-(2-thienyl)-ethyl]-amin und sein Hydrochlorid.

18. Di-[2-(3-thienyl)-ethyl]-amin und sein Hydrochlorid.

**Claims**

1. A process for the preparation of thienylethylamines of the formula

R—NH—R'

in which R represents

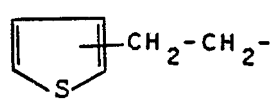

and R' represents H or the radical R, characterised in that a solution of a thienylacetonitrile of the formula

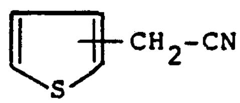

is hydrogenated in the presence of a nickel- or cobalt-based catalyst, at a pressure between $10^5$ and $10^7$ Pa and at a temperature between 15°C and 80°C.

2. A process according to claim 1, characterised in that the reaction is effected in an aliphatic alcohol.

3. A process according to claim 1 or 2, characterised in that the catalyst is Raney nickel.

4. A process according to claim 3, characterised in that the concentration of the nitrile in the reaction medium is between 20 and 40% (w/v) and that of the catalyst is between 2 and 30% by weight relative to the substrate.

5. A process according to any one of the claims 1 to 4, characterised in that the pressure is between 1 and 5 MPa.

6. A process according to any one of the claims 3 to 4, characterised in that the hydrogenation is effected in the presence of an inorganic or organic base.

7. A process according to claim 6, characterised in that the base is chosen from NaOH, LiOH and $K_2CO_3$.

8. A process according to claim 6, characterised in that the base is chosen from $NH_3$, KOH, $CS_2CO_3$ and $HON(R_1)_4$ in which $R_1$ represents a $C_1$ to $C_4$ alkyl.

9. A process according to any one of the claims 6 to 8, characterised in that the base is used in quantities varying from 0.1 to 15% relative to the quantity of thienyl-acetonitrile used.

10. A process according to any one of the claims 1 to 3, characterised in that the catalyst is URUSHIBARA cobalt, which is cobalt precipitated from a solution of one of its salts by an electropositive metal.

11. A process according to claim 10, characterised in that the pressure is between 1 and 6 MPa.

12. A process according to claim 1, characterised in that the reaction medium is acidified with a solvent.

13. A process according to claim 12, characterised in that the solvent is a mixture of a $C_1$—$C_4$ aliphatic alcohol and acetic acid.

14. A process according to claim 13, characterised in that the catalyst is chosen from Raney nickel and nickel boride.

15. A process according to one of the claims 1 to 14, characterised in that the primary and secondary amines are separated by distillation.

16. A process according to any one of the claims 1 to 14, characterised in that the primary and secondary amines are separated by precipitation of the hydrochloride of the secondary amine in an aqueous medium.

17. Di[2-(thien-2-yl)ethylamine and its hydrochloride.

18. Di[2-(thien-3-yl)ethylamine and its hydrochloride.